(19) Europäisches Patentamt
European Patent Office
Office européen des brevets

(11) Veröffentlichungsnummer: **0 270 481 B1**

# (12) EUROPÄISCHE PATENTSCHRIFT

(45) Veröffentlichungstag der Patentschrift: **17.04.91**

(51) Int. Cl.5: **C07C 405/00**, C07F 7/18, C07D 319/08, C07C 47/575

(21) Anmeldenummer: **87730143.2**

(22) Anmeldetag: **12.11.87**

## (54) Neues Verfahren zur Herstellung von optisch aktiven Carbacyclin-Zwischenprodukten.

(30) Priorität: **13.11.86 DE 3638757**

(43) Veröffentlichungstag der Anmeldung: **08.06.88 Patentblatt 88/23**

(45) Bekanntmachung des Hinweises auf die Patenterteilung: **17.04.91 Patentblatt 91/16**

(84) Benannte Vertragsstaaten: **AT BE CH DE ES FR GB GR IT LI LU NL SE**

(56) Entgegenhaltungen:
**US-A- 3 891 676**

**CHEMISTRY LETTERS, Nr. 3, März 1983, Seiten 385-388, The Chemical Society of Japan, Tokyo, JP; T. MUKAIYAMA et al.: "An asymmetric synthesis of bicyclic lactones and its application to the asymmetric synthesis of (1R,3S)-cis-chrysanthemic acid"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Skuballa, Werner, Dr.**
**Olwenstrasse 25**
**W-1000 Berlin 28(DE)**
Erfinder: **Dahl, Helmut, Dr.**
**Gollanczstrasse 102**
**W-1000 Berlin 28(DE)**

**EP 0 270 481 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung optisch aktiver Carbacyclin-Zwischenproduckte aus racemischen cis-Bicyclo[3.3.0]-octan-2-carbonsäuren. Mit diesem Verfahren lassen sich in vorteilhafter Weise wichtige Zwischenstufen für die Synthese von stabilen und pharmakologisch wirksamen Carbacyclinderivaten herstellen.

Die unterschiedlichen Synthesemöglichkeiten von Carbacyclinanaloga sind in den beiden folgenden Übersichtsartikeln zusammengefaßt: R.C. Nickolson, M.H. Town und H. Vorbrüggen, Medicinal Research Review 5 , 1 (1985); P.A. Aristoff in Advances in Prostaglandin, Thromboxane and Leukotriene Research, Vol. 15 (1985). Bisher waren alle bekannten Syntheseverfahren von reinen, optisch aktiven Carbacylinen mit einem erheblichen synthetischen Aufwand verbunden. So werden z.B. in DE-OS 2912409 und in DE-OS 3021895 Carbacyclinsynthesen beschrieben, die aus dem optisch aktiven "Corey-Lacton" nur unter großen Ausbeuteverlusten die Umwandlung in Carbacyclinzwischenstufen ermöglichen.

Es wurde nun gefunden, daß sich die diastereomeren Amide der Formel II, die aus D-(-)-α-Phenylglycinol und racemischen cis-Bicyclo[3.3.0]octan-2-carbonsäuren gebildet werden, überraschenderweise besonders gut trennen lassen und somit nach reduktiver Spaltung des Amids zur Herstellung der reinen, optisch aktiven Carbacyclinzwischenstufen der Formel 1 geeignet sind.

Die Erfindung betrifft somit ein Verfahren zur Herstellung von optisch aktiven cis-Bicyclo[3.3.0]octan-2-aldehyden der Formel I

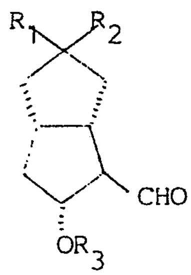

(I),

worin

$R_1$ und $R_2$ gemeinsam den 2-bindigen Rest -O-X-O mit X als geradkettigem oder verzweigtkettigem Alkylen mit 1-7 C-Atomen oder $R_1$ und $R_2$ jeweils den Rest OR mit R als geradkettigem oder verzweigtkettigem Alkyl mit 1-7 C-Atomen und

$R_3$ Wasserstoff, einen gesättigten Alkylrest mit 1-7 C-Atomen, einen Aralkylrest mit 7-10 C-Atomen, einen Tetrahydropyranylrest, einen Trialkylsilylrest mit 1-6 C-Atomen im alkyl, wobei alkyl auch mit Phenyl substituiert sein kann, einen Dialkylphenylsilylrest oder Alkyldiphenylsilylrest mit 1-16 C-Atomen im alkyl, bedeuten, das dadurch gekennzeichnet ist, daß man die diastereomeren Amide der Formel II

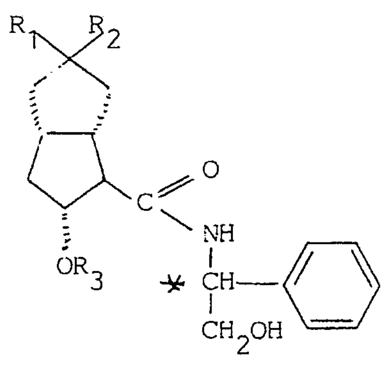

(II),

worin

$R_1$, $R_2$, und $R_3$ die oben genannten Bedeutungen haben, trennt und das enantiomerisch reine Amid der Formel II reduziert.

Wenn X einen geradkettigen oder verzweigtkettigen Alkylenrest mit 1-7 C-Atomen bedeutet, so sind damit folgende Reste gemeint:

$-(CH)_{2n}-$,mit n = 1-7 (Methylen, Ethylen, Tri-, Tetra-, Penta-, Hexa- und Heptamethylen), $-C(CH_3)_2-$, -CH-

$(CH_3)$-, $-CH((CH_3)-CH_2-$, $-C(CH_3)_2-CH_2-$, $-CH_2-CH(CH_3)-$, $C_2-C(CH_3)_2$, $-CH_2-CH(CH_3)-CH_2-$, $-CH_2-C(CH_3)_2-CH_2-$, $-CH-(C_2H_5)-$, $-C(C_2H_5)_2-$, $-CH(C_2H_5)-CH_2-$, $-C(C_2H_5)_2-CH_2-$, $-CH_2-CH(C_2H_5)-$, $-CH_2-C(C_2H_5)_2-$, $-CH_2-CH(C_2H_5)-CH_2-$, $-CH_2-C(C_2H_5)_2-CH_2-$ usw.

R als geradkettiger oder verzweigtkettiger Alkylrest mit 1-7 C-Atomen bedeutet Methyl, Ethyl, n-Propyl, Isopropyl, n-Butyl, Isobutyl, sek.-Butyl, tert.-Butyl, n-Pentyl, Isopentyl, sek.-Pentyl, Neopentyl, n-Hexyl, Isohexyl, n-Heptyl, Isoheptyl.

Mit $R_3$ bzw. $R_4$ (in Formel IV) als gesättigte Alkylreste mit 1-7 C-Atomen sind die für R bereits genannten Alkylreste gemeint.

$R_3$ bzw. $R_4$ als Aralkylreste mit 7-10 C-Atomen sollen folgende Reste umfassen:

$-CH_2-C_6H_5$, $-CH_2-CH_2-C_6H_5$, $-CH_2-CH_2-CH_2-C_6H_5$, $-CH_2-CH_2-CH_2-CH_2-C_6H_5$, $-CH(CH_3)-C_6H_5$, $-CH_2-CH(CH_3)-C_6H_5$, $-CH_2-CH_2-CH(CH_3)-C_6H_5$, $-CH(CH_3)-CH_2-C_6H_5$, $-CH(CH_3)-CH_2-CH_2-C_6H_5$, $-CH_2-CH(CH_3)-CH_2-C_6H_5$, $-CH(C_2H_5)-C_6H_5$, $-CH_2-CH(C_2H_5)-C_6H_5$, $-CH(C_3H_7)-C_6H_5$, $-C(CH_3)_2-C_6H_5$, $-CH_2-C(CH_3)_2-C_6H_5$, $-CH(CH_3)-CH(CH_3)-C_6H_5$, $-C(CH_3)_2-CH_2-C_6H_5$ usw.

Für die "alkyl"-Reste in Trialkyl- (mit 1-6 C-Atomen im alkyl), Dialkylphenyl-oder Alkyldiphenyl-silyl mit 1-16 C-Atomen im alkyl kommen die für R bereits genannten Reste in Betracht.

Die Verbindungen der Formel I eignen sich besonders als Zwischenprodukte für die Herstellung von stabilen, pharmakologisch wirksamen Carbacyclinderivaten (z.B. Iloprost).

Die Herstellung der Amide der Formel II erfolgt nach den dem Fachmann bekannten Methoden. Beispielsweise kann die Herstellung der diastereomeren Amide der Formel II durch Umsetzung der racemischen Carbonsäuren der Formel III

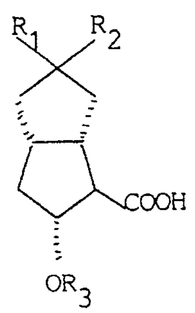

(III),

worin $R_1$, $R_2$ und $R_3$ die oben genannten Bedeutungen haben, mit einer Base, vorzugsweise Triäthylamin, und Chlorameisensäureisobutylester zum intermediären Anhydrid und anschließender Zugabe von D-(-)-α-phenylglycinol erfolgen. Die Reaktion wird bei -20°C bis 80°C, vorzugsweise 0°C bis 30°C in einem inerten Lösungsmittel durchgeführt. Als Lösungsmittel können beispielsweise Aceton, Acetonitril, Methylenchlorid, Äthylenchlorid u.a. verwendet werden. Die Trennung der diastereomeren Amide kann in einfacher Weise durch Säulenchromatographie an Kieselgel oder Aluminiumoxid oder durch fraktionierte Kristallisation erfolgen.

Als Reduktionsmittel für die Amidspaltung kann beispielsweise Diisobutylaluminiumhydrid dienen. Die reduktive Amidspaltung wird bei -120°C bis 30°C, vorzugsweise -70°C bis 0°C durchgeführt. Als Lösungsmittel oder als Lösungsmittelgemische können beispielsweise Toluol, Tetrahydrofuran, Diäthyläther, Methylenchlorid, Athylenchlorid u.a. verwendet werden.

Das für das erfindungsgemäße Verfahren benötigte Ausgangsmaterial der Formel III läßt sich aus dem bekannten cis-Bicyclo[3.3.0]octan-3,7-dion durch selektive Ketalisierung mit einem der Bedeutung von $R_1$ und $R_2$

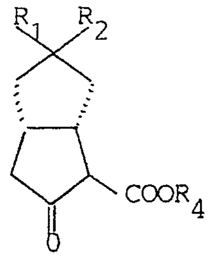

(IV),

entsprechenden Alkohol oder Diol herstellen. Einführung der $COOR_4$ Gruppe durch Umsetzung beispielsweise mit Basen und Dialkylcarbonaten zu der Verbindung der Formel IV, worin $R_4$ für Alkyl mit 1-7 C-Atomen oder Aralkyl mit 7-10 C-Atomen steht, sowie anschließende Reduktion der Ketogruppe mit Natriumborhydrid und gegebenenfalls Schutz der erzeugten Hydroxygruppe z.B. durch Silylierung mit tert.-Butyldimethylsilylchlorid, und Verseifung der Estergruppe führt zum Ausgangsmaterial der Formel III. Die weitere Umwandlung der Verbindungen der Formel I in die pharmakologisch wirksamen Carbacyclinderivate erfolgt nach bekannter Verfahrensweise, beispielsweise durch Kondensation mit 3-Methyl-2-oxo-hept-5-in-phosphonsäuredimethylester und Natriumhydrid zum α, β-ungesättigten Keton 5 . Reduktion des Ketons 5 zum Alkohol 6 , anschließende Schutzgruppenbabspaltung zum Diol 7 und Tetrahydropyranylätherbildung lieferte das Keton 8 , welches nach Wittigreaktion mit dem Ylen aus 4-Carboxybutyltriphenylphosphoniumbromid und anschließender Schutzgruppenabspaltung mit wäßriger Essigsäure in das Carbacyclinderivat Iloprost überführt wird.

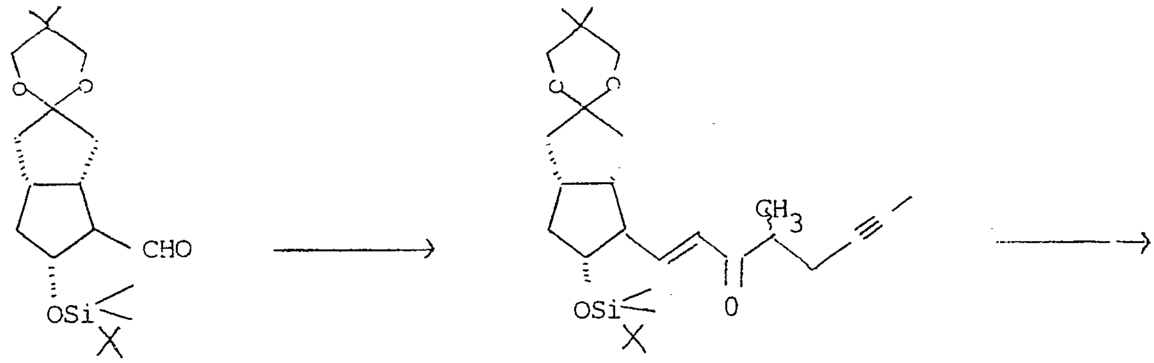

I ($R_1$ und $R_2$= $-O-CH_2-C(CH_3)_2-CH_2-O-$,
$R_3$ = Dimethyl-tert.butylsilyl)

5

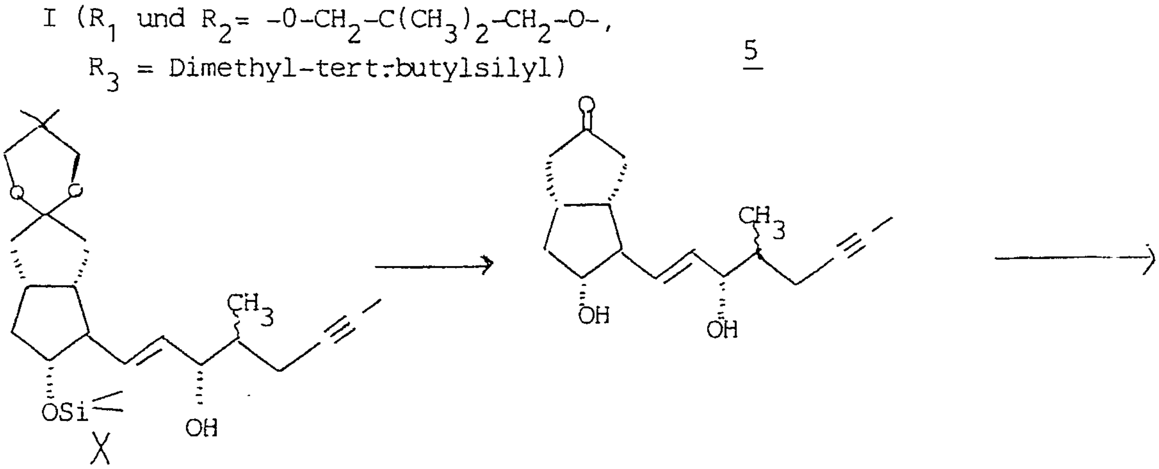

6

7

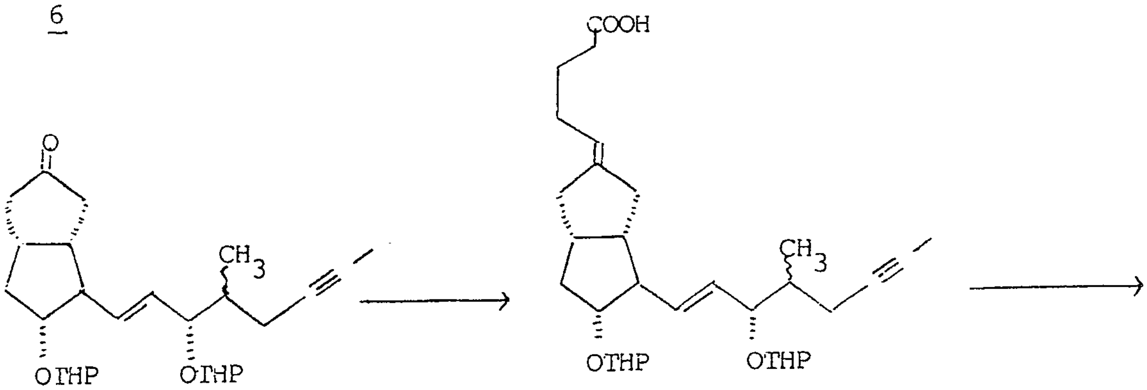

8

9

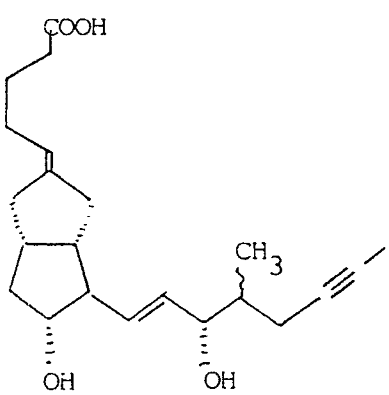

Iloprost

Beispiel 1

**7,7-(2,2-Dimethyl-trimethylendioxy)-2β-formyl-3α-(tert.-butyldimethylsilyloxy)-cis-bicyclo[3.3.0]octan**

Zu einer Lösung von 4,8 g 7,7-(2,2-Dimethyltrimethylendioxy)-3α-(tert.-butyldimethylsilyloxy)-cis-bicyclo[3.3.0]octan-2β-carbonsäure in 55,6 ml Aceton fügt man bei 0 °C 2,08 ml Triäthylamin, rührt 5 Minuten, versetzt mit einer Lösung aus 1,92 ml Chlorameisensäureisobutylester in 35 ml Aceton und rührt 20 Minuten bei 0 °C. Anschließend tropft man eine Lösung von 1,88 g D(-)α-Phenylglycinol in 17 ml Aceton und 17 ml Acetonitril zu und rührt 24 Stunden bei 25 °C. Man engt im Vakuum ein, nimmt in 200 ml Methylenchlorid auf, schüttelt mit Sole, trocknet über Magnesiumsulfat und dampft im Vakuum ein. Den Rückstand trennt man durch Säulenchromatographie an Kieselgel mit Essigester/Hexan (6:4). Dabei erhält man als unpolare Substanz nach Umkristallisation aus Toluol 2,7 g des D(-)α = Phenylglycinolamids mit der durch die allgemeine Formel II wiedergegebenen absoluten Konfiguration. (Schnmelzpunkt 157-159 °C). (-$[\alpha]_D$ = -25,9 ° (c = 0,54 in $CHCl_3$)).

Als polare Komponente erhält man nach weiterer Elution mit EtOAc/Hexan ( 1 : 1 ) Abdampfen und anschließender Umkristallisation aus Toluol 2,25 g des entsprechend spiegelbildlich konfigurierten D(-)α-Phenylglycinolamids (Schmelzpunkt 148-149 °C). ($[\alpha]_D$ = -9,3 ° (c = 0,32 in $CHCl_3$)). Daneben werden noch Mischfraktionen erhalten, die sich leicht durch nochmalige Chromatographie oder fraktionierte Kristallisation trennen lassen.

Die Zuordnung der absoluten Konfigurationen erfolgt durch Schmelzpunkt-, NMR-und Dünnschichtvergleich der nach Ketal- und Silylätherspaltung erhaltenen Amide mit dem entsprechenden Amid, welches aus Zwischenprodukten bekannter absoluter Konfiguration zu Vergleichszwecken synthetisiert wurde.

Zur reduktiven Amidabspaltung zu dem Aldehyd der allgemeinen Formel I löst man 1,5 g des vorstehend erhaltenen unpolaren D(-)α-Phenylglycinolamids in 20 ml Methylenchlorid und tropft langsam 12,3 ml einer 14,8 molaren Lösung von Diisobutylaluminiumhydrid in Toluol. Man rührt 1 Stunde bei -70 °C, tropft 4 ml Isopropanol zu, fügt 4 ml Wasser zu, verdünnt mit 10 ml Methylenchlorid, rührt 1 Stunde bei 25 °C, filtriert und dampft im Vakuum ein. Den Rückstand reinigt man durch Säulenchromatographie an Kieselgel. Mit Essigester-Hexan (3 + 2) erhält man 444 mg der Titelverbindung als farbloses Öl.

IR ($CHCl_3$): 2960, 2860, 2720,1719, 840 $cm^{-1}$.

Der Drehwert beträgt $[\alpha]_D^{20}$ -23,6 ° (c = 0,95 in $CHCl_3$).

Daneben wurden noch 530 mg des unumgesetzten Ausgangsamids isoliert.

Das Ausgangsmaterial für die obige Titelverbindung wurde wie folgt hergestellt:

1a) (2,2-Dimethyl-trimethylendioxy)-cis-bicyclol[3.3.0]octan-3-on-2-carbon säuremethylester

Man suspendiert 38,34g 55-60%iges Natriumhydrid in 616 ml Dimethylcarbonat, erwärmt unter Stickstoff auf 50 °C und gibt eine kleine Menge der Lösung von 49,31 g 3,3-(2,2-Dimethyltrimethylendioxy)-cis-bicyclo[3.3.0] octan-7-on in 370 ml Dimethylcarbonat dazu. Durch Zugabe von 2 ml Methanol induziert man die Reaktion, gibt die restliche Lösung dazu und rührt insgesamt 7,5 Std. bei 50 °C. Man kühlt im Eisbad, zersetzt überschüssiges Natriumhydrid mit Methanol, gibt Wasser hinzu und neutralisiert mit Essigsäure. Man extrahiert das Produkt mit Dichlormethan, konzentriert im Vakuum und kristallisiert das Produkt mit Hexan. Man erhält 53,44 g Produkt vom Schmelzpunkt 72 °C.

1b) 7,7-(2,2-Dimethyl-trimethylendioxy)-3α-hydroxy-cis-bicyclo[3.3.0]-octan,2β-carbonsäuremethylester

**Methode A**

Man löst 52,0 g 7,7-(2,2-Dimethyl-trimethylendioxy-cis-bicyclo[3.3.0] octan-3-on-2-carbonsäuremethylester in der Wärme in 1000 ml Methanol und kühlt auf -40 °C ab. Dann trägt man 20,91 g Natriumborhydrid ein, rührt 30 Minuten, versetzt langsam mit 171 ml Aceton und neutralisiert nach einer weiteren Stunde mit Essigsäure. Nach Abdestillieren der Hauptmenge Lösungsmittel wird mit Wasser und Dichlormethan versetzt, die organische Phase mit Natriumsulfat getrocknet und im Vakuum konzentriert. Den Rückstand nimmt man in 550 ml Methanol auf, fügt 9,94 g Natriummethylat dazu und erwärmt 105 Minuten auf 40 °C. Man kühlt im Eisbad, neutralisiert und arbeitet wie vorher beschrieben auf. Das erhaltene Rohprodukt chromatographiert man an Kieselgel mit Dichlormethan-Ethylacetat-Gemischen. Man erhält 47 g der

gewünschten Verbindung, die sich mit Hexan kristallisieren läßt und einen Schmelzpunkt von 43°C aufweist.

**Methode B**

56,6 g 7,7-(2,2-Dimethyl-trimethylendioxy)-cis-bicyclo[3.3.0]octan-3-on-2-carbonsäuremethylester werden in 300 ml Ethylacetat gelöst und nach Zugabe von 5,1 g Platindioxid bei 22°C unter Normaldruck hydriert, bis die Wasserstoffaufnahme beendet ist. Man filtriert vom Katalysator und konzentriert im Vakuum. Man erhält 56,8 g der gewünschten Verbindung als Rohprodukt. Mann kann das Produkt aus Hexan kristallisieren und erhält 44,4 g Erstkristallisat vom Schnmelzpunkt 43°C.

1c) 7,7-(2,2-Dimethyl-trimethylendioxy)-3α-tert.-butyldimethylsilyloxy-cis-bicyclo[3.3.0.]octan-2β-carbonsäuremethylester

Man löst 8,53 g 7,7-(2,2-Dimethyl-trimethylendioxy)-3α-hydroxy-cis-bicyclo[3.3.0]octan-2β-carbonsäuremethylester in 9 ml Dimethylformamid, fügt 5,11 g Imidazol und 5,43 g tert.-Butyl-dimethylchlorsilan hinzu und rührt 1,5 Std. bei 22°C. Man versetzt mit Diethylether und Natriumchlorid-Lösung, wäscht die Etherphase mit kalter, 1-normaler Schwefelsäure, Natriumhydrogencarbonat-Lösung und Natriumchlorid-Lösung, trocknet mit Natriumsulfat und konzentriert im Vakuum. Man erhält 12, 17 g Produkt, das für die weitere Umsetzung ausreichend rein ist.

1d) 7,7-(2,2-Dimethyl-trimethylendioxy)-3α-tert.-butyldimethylsilyloxy-cis-bicyclo[3.3.0]octan-2β-carbonsäure

Man löst 9,54 g 7,7-(2,2-Dimethyl-trimethylendioxy)-3α-tert.-butyldimethylsilyloxy-cis-bicyclo[3.3.0]-octan-2β-carbonsäuremethylester in 50 ml Methanol und 26 ml 5%iger Natronlauge und erhitzt 1,5 Std. unter Rückfluß. Man konzentriert im Vakuum, verdünnt mit Wasser und entfernt Neutralstoffe durch Extraktion mit Diethylether. Man kühlt im Eisbad, versetzt mit 2-normaler Schwefelsäure bis pH = 3 erreicht ist, extrahiert mit Diethylether, trocknet mit Calciumsulfat und konzentriert im Vakuum. Man erhält 9,2 g kristallines Produkt, das für die weitere Umsetzung ausreichend rein ist.

## Ansprüche

1. Verfahren zur Herstellung von optisch aktiven cis-Bicyclo[3.3.0]octan-2-aldehyden der Formel I

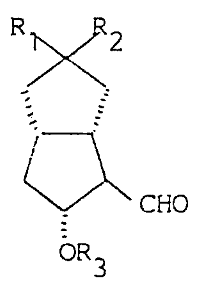

(I),

worin
$R_1$ und $R_2$ gemeinsam den 2-bindigen Rest -O-X-O mit X als geradkettigem oder verzweigtkettigem Alkylen mit 1-7 C-Atomen oder $R_1$ und $R_2$ jeweils den Rest OR mit R als geradkettigem oder verzweigtkettigem Alkyl mit 1-7 C-Atomen und
$R_3$ Wasserstoff, einen gesättigten Alkylrest mit 1-7 C-Atomen, einen Aralykylrest mit 7-10 C-Atomen, einen Tetrahydropyranylrest, einen Trialkylsilylrest mit 1-6 C-Atomen im alkyl, wobei alkyl auch mit Phenyl substituiert sein kann, einen Dialkylphenylsilylrest oder Alkyldiphenylsilylrest mit 1-16 C-Atomen im alkyl, bedeuten, das dadurch gekennzeichnet ist, daß man die diastereomeren Amide der Formel II

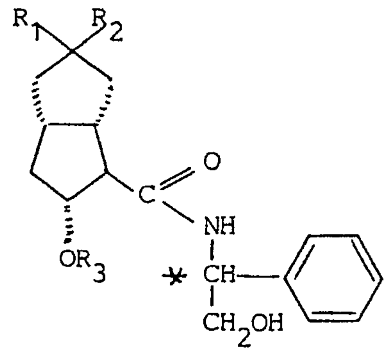

(II),

worin
$R_1$, $R_2$, und $R_3$ die oben genannten Bedeutungen haben, trennt und das enantiomerisch reine Amid der Formel II reduziert.

## Claims

1. Process for the preparation of optically active cis-bicyclo[3,3,0]octane-2-aldehydes of formula I

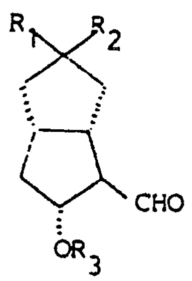

(I)

in which
$R_1$ and $R_2$ together represent the two-bonded radical -O-X-O- wherein X is a straight-chain or branched alkylene radical having from 1 to 7 carbon atoms, or each of $R_1$ and $R_2$ represents an OR radical wherein R is a straight-chain or branched alkyl radical having from 1 to 7 carbon atoms, and
$R_3$ represents hydrogen, a saturated alkyl radical having from 1 to 7 carbon atoms, an aralkyl radical having from 7 to 10 carbon atoms, a tetrahydropyranyl radical, a trialkylsilyl radical having from 1 to 6 carbon atoms in the alkyl moiety, wherein alkyl may also be substituted by phenyl, a dialkylphenylsilyl radical or alkyldiphenylsilyl radical having from 1 to 16 carbon atoms in the alkyl moiety, characterised in that the diastereoisomeric amides of formula II

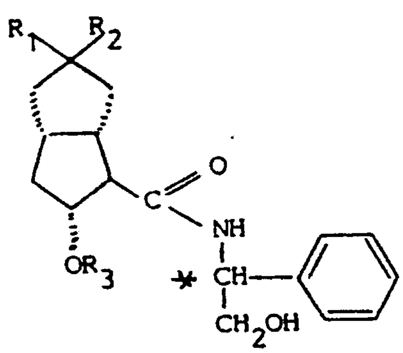

(II),

in which
$R_1$, $R_2$ and $R_3$ have the meanings given above, are separated and the enantiomerically pure amide of formula II is reduced.

## Revendications

1. Procédé pour préparer des bicyclo[3.3.0]-octane-carbaldéhydes-2 cis optiquement actifs qui répondent à la formule I :

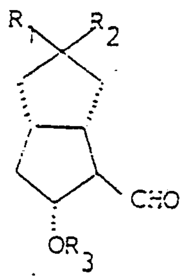

(I).

dans laquelle :

$R_1$ et $R_2$ forment ensemble un radical bivalent -O-X-O- dans lequel X désigne un alkylène, linéaire ou ramifié, contenant de 1 à 7 atomes de carbone, ou $R_1$ et $R_2$ représentent chacun un radical -OR dans lequel R représente un alkyle, linéaire ou ramifié, contenant de 1 à 7 atomes de carbone, et $R_3$ représente l'hydrogène, un radical alkyle saturé contenant de 1 à 7 atomes de carbone, un radical aralkyle contenant de 7 à 10 atomes de carbone, un radical tétrahydropyrannyle, un radical trialkylsilyle dont chacun des alkyles contient de 1 à 6 atomes de carbone et peut porter un phényle comme substituant, ou un radical dialkylphénylsilyle ou alkyldiphénylsilyle dont l'alkyle ou chacun des alkyles contient de 1 à 16 atomes de carbone, procédé caractérisé en ce qu'on sépare les amides diastéréoisomères répondant à la formule II :

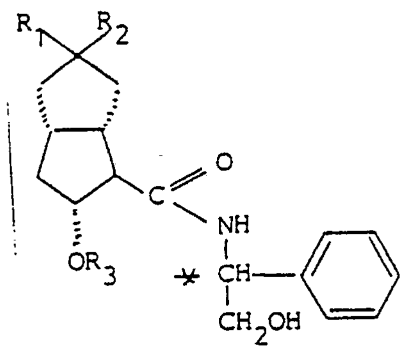

(II)

dans laquelle $R_1$, $R_2$ et $R_3$ ont les significations qui leur ont été données ci-dessus, et on réduit l'amide chiralement pur de formule II.